# EUROPEAN PATENT APPLICATION

(11) **EP 3 078 746 A1**
(43) Date of publication of application: **12.10.2016**
(21) Application number: 14839532.0
(22) Date of filing: 25.08.2014
(51) Int. Cl.: C12N 15/115, C07H 21/04, C12N 5/0775

(54) **CHEMICALLY MODIFIED POLYNUCLEOTIDES AND METHOD FOR PRODUCING CHEMICALLY MODIFIED POLYNUCLEOTIDES**

(30) Priority: 26.08.2013 BR 102013021701
(71) Applicant: Universidade de São Paulo, 05347-902 São Paulo - SP (BR)
(72) Inventor: ULRICH, Alexander Henning, 06713460 Cotia (BR); BASSANEZE, Vinícius, 05579000 São Paulo (BR); NERY, Arthur Andrade, 05588000 São Paulo (BR); KRIEGER, José Eduardo, 01230010 São Paulo (BR)
(74) Representative: Carvajal y Urquijo, Isabel
(86) International application number: PCT/BR2014/000294
(87) International publication number: WO 2015/027305

(57) **Abstract**

The present invention relates to chemically-modified polynucleotides of formula (I): 5'-CONS-SEQ.ID.n-CONS-3' (I) containing one or more modified pyrimidines and at least one inverted nucleotide repeat, and to the method for producing the same.

## Description

### FIELD OF THE INVENTION

This invention relates to the field of compounds and preparations containing active organic ingredients; specifically those compounds containing polynucleotides containing modified bases for cell identification.

### PRIOR ART

The pharmaceutical industry and biotechnology companies have used various combinatorial chemistry techniques as a tool for identifying new materials with specific properties.

This tool is characterized by the simultaneous synthesis and analysis of large libraries consisting of compounds with related molecular formulas but structurally different, and the probability of success in the identification of functional molecules grows with the diversity of the library (Stoltenburg et al., 2007, Ulrich and Trujillo, 2008).

The polynucleotides are attractive compounds for combinatorial chemistry, since they fold into defined secondary and tertiary structures and can be amplified by enzymatic synthesis.

Polynucleotide libraries with random sequences with approximately 10¹⁵ different molecules can be obtained by chemical synthesis, and then used for the identification of various compounds for different purposes, such as high affinity binding to a particular target (polynucleotides of formula (I)) or catalytic activity (ribozymes and DNAzymes) (Stoltenburg et al., 2007, Ulrich and Trujillo, 2008).

In 1990, a technique was described for selecting, from combinatorial libraries of oligonucleotides (DNA or RNA), molecules that bind with high affinity and specificity to certain molecular targets. This technique called SELEX (Systematic Evolution of Ligands by Exponential enrichment) was developed simultaneously and independently by Tuerk and Gold and Ellington and Szostak (Tuerk and Gold. 1990 Ellington and Szostak, 1990) (US Pat. No. 5,475,096, Gold and Tuerk, 1995; US Pat. No. 5,270,163, Gold and Tuerk, 1993)).

The functional molecules identified in this procedure are called "polynucleotides of formula(I)" (from Latin, *aptus* which means "fit, suitable, adjusted," and the Greek, which means merely "one of the constituent parts of a whole, particle") (Ellington and Szostak, 1990).

The SELEX technique has been used to select polynucleotides of formula (I) of RNA and DNA from a wide variety of targets, including: ions (Ciesiolka etal., 1995), nucleotides (Sassanfar and Szostak, 1993), nucleic acids (Ko et al., 1999), carbohydrates (Yang et al., 1998), amino acids (Geiger et al., 1996), peptides (Bock et al., 1992), antibiotics (Berens et al., 2001), complex targets such as membrane receptors (Ulrich et al., 1998, 2002) and whole cells (Wang et al., 2003, Homannand Goringer, 1999), among others.

Several polynucleotides of formula (I) identified bind to their targets with similar specificity and affinity of monoclonal antibodies with affinity constants near nano- and femto mole, being able, for example, to differentiate ATP from dATP (Sassanfar and Szostak, 1993) or to discriminate PC12 cells differentiated in parental PC12 cell neurons (Wang et al., 2003).

Polynucleotides of formula (I) as ligands or protein inhibitors are based on the property of DNA and RNA molecules to recognize specific protein epitopes, similar to the interaction RNA-protein and DNA-protein existing in the cell.

Three-dimensional structures formed by polynucleotides of formula (I), based on their specific sequences which, in turn, lead to unique structures, when complexed to their ligands, showed that the specificity and binding affinity to their targets are conferred by the combination of the following properties: structural compatibility, precise stacking of aromatic rings, van der Waals and electrostatic interactions, and finally, formation of hydrogen bridges.

In the presence of the target, the formation of the binding complex, adaptive aptamer undergoes conformational changes. The folding into a defined three-dimensional structure allows the aptamer to completely encapsulate small substances for the generation of a specific binding pocket.

For molecules with high molecular weight such as proteins, different target surface structures are involved in the interaction with the aptamer (Hermann and Patel, 2000). In the last 20 years, research and development of the polynucleotides of formula (I) demonstrate their potential as a new and effective class of therapeutic molecules.

The sequence of these ligands, especially with the potential biomedical applications, optimizations in their selection protocols, as well as chemical modifications which enhance their stability and pharmacokinetics have been protected by patents. One of these polynucleotides of formula (I), the pharmaceutical product Macugen™ (pegaptanib sodium injection from Pfizer, Eyetech Pharmaceuticals), which antagonizes efficient and selective action of the isoform 165 of the endothelial vascular growth factor (VEGF) was the first aptamer to be approved in 2004 by FDA (US Food and Drug Administration) for the treatment of macular degeneration related to aging (Bell et al., 1999, Eyetech Study Group, 2002, Ishida et al., 2003, Majumder et al., 2009).

Several patents describe the use the SELEX technology. Document US7179894 shows the production of polynucleotides of formula (I) resistant to nucleases, which may allow the production of these molecules in vivo applications such as the use of polynucleotides of formula (I) for the treatment of diseases, for diagnostic use and even validation of therapeutic targets, as described in document US20070066551.

With this technological base, there is the development of polynucleotides of formula (I) which bind with high affinity to plasma proteins such as, for example, thrombin, a process described in document US7998939B2 and which, if used as a therapeutic means could be expressed in cells for the production and secretion in specific locations as shown in document US7700759.

Specifically, using the Cell-SELEX technique, document US20090239762 shows the production of polynucleotides of formula (I) for identifying lung cancer cells, targeting both the diagnosis and use of these molecules for future therapies.

Therefore, the prior art lacks information regarding polynucleotides or DNA sequences with specific sequences or involved in exogenous processes of cell purification, in this case, mesenchymal adipose tissue stem cells, including modified polynucleotides of formula (I).

### OBJECT OF THE INVENTION

The present invention aims to provide chemically-modified polynucleotides of formula (I): 5'-CONS SEQ.ID.n-CONS-3', capable of identifying and isolating stem cells from the adipose tissue such as, for example, from human lipoaspirate.

It is a further object of the present invention the process for the production of chemically-modified polynucleotides of formula (I):

5'-CONS-SEQ.ID.n-CONS-3'.

### SUMMARY OF THE INVENTION

The present invention relates to a family of 32 chemically-modified polynucleotides sequences of formula (I) by the addition of biotin or fluorescent probe in the terminal region 5', being relevant in any process involving the identification, purification, and concentration of mesenchymal stem cells, including, but not limited to its use as a reagent in pharmacological assays, for example, in vivo and in vitro assays of cytomics in diagnostic tests and as a high affinity ligand for cell enrichment for subsequent therapeutic applications such as, for example, regenerative therapy of bone diseases, arteriosclerosis, cardiovascular ischemia or brain transplantation and in neurodegenerative diseases such as Parkinson's and amyotrophic lateral sclerosis and others.

All polynucleotides of formula (I) in the present invention were characterized, and those of SEQ.ID.9 and SEQ.ID.II demonstrated identification of 16 and 23%, respectively, of cells with exclusive signatures among the so-called pure initial population of mesenchymal stem cells from lipoaspirate.

### BRIEF DESCRIPTION OF THE DRAWINGS

To obtain a complete view of the objectives of the present invention, this document shall be read together with the accompanying drawings and to which reference is as follows.
Figure 1: Classes of sequences present inR12 and BR5 libraries.
Figure 2: Structures and sequences of Class I of polynucleotides of formula (I) found in the sequencing of BR5 and R12 liabraries.
Figure 3: Structures and sequences of Class II of polynucleotides of formula (I) found in the sequencing of BR5 and R12 libraries.
Figure 4: Structures and sequences of Class III of polynucleotides of formula (I) found in the sequencing of BR5 and R12 libraries.
Figure 5: Structures and sequences of Class IV of polynucleotides of formula (I) found in the sequencing of BR5 and R12 libraries.
Figure 6: Structures and sequences of Class V of polynucleodies of formula (I) found in the sequencing of BR5 and R12 libraries.
Figure 7: Structures and sequences of polynucleotides of formula (I) found in the sequencing of BR5 and R12 libraries including a control sequence with random region composed of a Poly-A sequence.
Figure 8: Characterization by cytometry with isolated polynucleotides of formula (I).
Figure 9: Characterization by cytometry with isolated polynucleotides of formula (I).

### DETAILED DESCRIPTION OF THE INVENTION

The present invention relates to chemically-modified polynucleotides of formula (I):

5'-CONS-SEQ.ID.n-CONS-3' (I),

wherein:
5' CONS is one of the identifying sequences SEQ. ID. 33 and or SEQ. ID. 34;
CONS-3 'is one of the identifying sequences SEQ. ID. 33 and/or SEQ. ID. 34;
SEQ.ID.n is alternately or separately one or more of identifying sequences of SEQ.ID. 1 to 32 containing one or more modified pyrimidines and at least one inverted nucleotide.

In the chemically-modified polynucleotides of formula (I)

5'-CONS-SEQ.ID.n-CONS-3' (I)

preferably:
5'-CONS is SEQ. ID. 33;
CONS-3' is SEQ. ID. 34;
SEQ.ID.n is separately each one of the identifying sequences of SEQ.ID. 1 to 32 containing one or more modified pyrimidine and last nucleotide inverted.

Modifications of one or more pyrimidines are made by replacing the 2-OH with a halogen atom.

Preferably, the modifications of one or more pyrimidines occur by substitution of 2'-R group of pyrimidine nucleotides with a fluorine atom. More preferably, the modified pyrimidines are 2'F-dCTP and 2'F-dCUTP.

Furthermore, the chemically-modified polynucleotides of formula (I) are altered in the 5' and/or 3' by adding markers belonging to the group consisting of: FAM, TET, HEX, TAMRA, ROX, CY3, CY3.5, Texas Red, CY5, Cy5.5, CY7, fluorescent compounds of the Alexa family and biotin.

Each of the chemically-modified polynucleotides of formula (I) containing one or more modified pyrimidines possess high affinity binding to proteins and cells and is therefore useful for detection, identification and purification of proteins and cells.

Therefore, the chemically-modified polynucleotides of formula (I) may be useful in the selection of proteins and cells, such as mesenchymal stem cells; preferably mesenchymal adipose tissue stem cells.

The second object of the present invention is a process for the production of chemically-modified polynucleotides of formula (I):
5'-CONS-SEQ.ID.n-CONS-3' (I)
which comprises the steps of:
   (a) Production of a polynucleotide library;
   (b) Presentation of polynucleotides to possible binding targets;
   (c) Selection and amplification of the ligands; and,
   (d) Modification of the ligands.

In step (a) the primer polynucleotides of sequences SEQ. ID. 33 and SEQ.ID. 34 are used to produce ssDNA library containing approximately10²⁰ polynucleotides encoding sequences containing a randomized region flanked by constant regions represented by polynucleotides that pair with primer sequences SEQ. ID. 33 and SEQ. ID. 34.

The chemically-modified polynucleotides so produced are identified and isolated by means known to those skilled in the art, such as measuring the size of chains by acrylamide gel and/or agarose gel, followed by purification with phenol/chloroform solution and others.

In step (b) the chemically-modified polynucleotides of formula (I) 5'-CONS-SEQ.ID.n -CONS 5'-3' are incubated in selection buffer and possible targets, and those that bind or remain bound after washing in each wash cycle are collected and go directly to the steps of enzymatic amplification of DNA by polymerase chain reaction (PCR). This reaction is designed to find molecules with higher affinity for the target of interest.

For the purposes of this invention, the possible targets of chemically-modified polynucleotides of formula (I) 5'-CONS-SEQ.ID.n-CONS-3' are organic molecules of molecular signature of the mesenchymal stem cell membranes. The PCR begins by denaturing the molecules collected for approximately 10 min at a temperature between 75 and 85°C, followed by renaturation for about 15 min at temperature between 20 to 25°C, then incubated with the target for a period of 4 from 0 to 60 minutes at a temperature of from 20 to 25°C.

The sequences with low or no affinity are to remain free in the selection buffer consisting of 145mm NaCl, 5. 3 mM KCI, 1.8 mM CaCl₂ *2H₂O, 1.7 mM MgCl₂ * 6H₂O and 25mM HEPES, pH adjusted from 7.0 to 7.5, while the others is associated to the target. The complex polynucleotide of formula (I)-target is separated from the free molecules by one of several processes available which include: adsorption on nitrocellulose filter, separation by gel-shift, capillary electrophoresis, among others.

The RNA or DNA molecules are eluted from their binding sites, collected and amplified by RT-PCR or PCR, respectively. The collection of resulting dsDNA, enriched with sequences with affinity for the target, is prepared for a new selection cycle.

For chemically-modified polynucleotides of formula (I) of DNA, dsDNA is denatured for purification of the sense strand used in the procedure and in the case of RNA, the library is generated by in vitro transcription.

Step (c) occurs at random, being carried out simultaneously from 15 to 30 polymerase chain reactions (PCR) each one between 0.5 to 2 pmol/µl of primer polynucleotide of SEQ. ID. 33, dideoxynuclotides and reagents known to those skilled in the art to amplify about l0 fmol of polynucleotides from the ssDNA library. The reaction occurs in cycles of 4 to 6 minutes at a temperature between 90 and 100°C; followed by 4 to 6 minutes at a temperature between 40 and 45°C and 10 minutes at 72°C. After the double strand synthesis, the primer of SEQ. ID. 34 is added, which confers a triple marker tail of biotin.

The amplification continues during 1 to 5 minutes at a temperature between 90° to 100°C to separate the tapes and cycles 30 to 60 seconds at a temperature between 90° and 100°C, followed by 30 to 60 seconds at a temperature between 40° to 50°C and a step from 1 to 5minutes at a temperature between 70° to 75°C and this cycle repeated 20 to 30 times before a final extension step of 5 to 12 minutes at a temperature between 70° to 75°C.

A sequence of the binding events to the target, selection and amplification is called SELEX cycle. These steps are repeated several times to stabilize the library affinity for its target.

After the process of selection and stabilization of affinities, the selected chemically-modified polynucleotides of formula (I) are amplified by PCR and cloned in bacterial vector, such as, for example, a vector as pGEM or the like following the protocol supplied by the manufacturer. Clones are isolated and individual chemically-modified polynucleotides of formula (I) are identified by sequencing and further characterized as to the target binding affinity and specificity.

Table 1 shows the chemically-modified polynucleotides of formula (I) 5'CONS-SEQ.ID.n-CONS-3', wherein SEQ.ID.n is independent and randomly a sequence from SEQ. SEQ. ID. 1 to SEQ. ID. 32 produced according to the process above, and indicates the base number of each single strand of DNA (ssDNA) and the presence of each of the sequences of SEQ. ID. 1 to SEQ. ID.

32 on ssDNA library made to identify polynucleotides able to recognize the molecular signature of mesenchymal stem cells membrane.

| **Nucleotideos** | **Indies Presença** | **Sequencia n** | **Nome** |
|---|---|---|---|
| 30 | 23,50 | GACCCAAGCCG**A**CCGCCCCCCATGCGTCGCCGTCTA | SEQ. ID. 1 |
| 33 | 0,39 | GAGGGCCGCCT**GGAA**CAATGGGTTCGGCGCGA | SEQ. ID. 2 |
| 34 | 0,36 | GCACGGACGGGCCGCA**C**GCGCTTG**A**GCCCGGGGA | SEQ. ID. 3 |
| 35 | 0,46 | GCAGGGGCGGGCCGC**ATA**CGCTTGAGCCCGGCGA | SEQ. ID. 4 |
| 34 | 0,43 | GCAGGGGCGCGGGC**ATG**CGCGCTTGAGCCCGGGGA | SEQ. ID. 5 |
| 34 | 48,00 | GCCGC**A**GGCGACGGTTCCCTTGAGTCGGGGTGGA | SEQ. ID. 6 |
| 35 | 0,26 | GCCG**AA**GGGCGTCGGAGCGCGCATCACAGGGGA | SEQ. ID. 7 |
| 35 | 0,26 | GCCGGGGGGCG**T**CGGAGGCGCCATAAGGGGCA | SEQ. ID. 8 |
| 35 | 0,30 | GCCGGGGCGTCGG**A**GCGCGCGTCCAGGGGGA | SEQ. ID. 9 |
| 34 | 0,31 | GGCGGGGGGGCCTGG**AA**GATGGGTTCGGCGCG**A** | SEQ. ID. 10 |
| 33 | 0,26 | GGCGGGGGCGCCGGAGCGCGCCATCGCAGGGGGA | SEQ. ID. 11 |
| 35 | 23,00 | GGGCAGGCGACCCGGTTCCCTT**GA**GTCGGGGTGG**A** | SEQ. ID. 12 |
| 33 | 0,29 | GGGGATCGCC**T**GGAACGATGGGTTCGGCGGACCA | SEQ. ID. 13 |

| **Nucleotideos** | **Indice Presença** | **Sequência n** | **Nome** |
|---|---|---|---|
| 33 | 0,31 | GGCCACCGCCCTGGAGCGATCGGGC**T**GTA | SEQ. ID. 14 |
| 34 | 0,31 | GGGCACCGCCCTGG**AG**CGATGGGC**TG**CA | SEQ. ID. 15 |
| 35 | 0,66 | GGGGCATCCCGCTGGACCCA**T**GGCGGATA | SEQ. ID. 16 |
| 35 | 0,63 | GGGCATCCCGC**TTG**GCACAATGCGTGAGCTGG**CA** | SEQ. ID. 17 |
| 35 | 0,68 | GGGGCATCCCGC**T**TGGCACAA**T**GCCCGGATA | SEQ. ID. 18 |
| 34 | 0,59 | GGGGCATCCCGCTTGGCACAATGCGCGCCAGGA | SEQ. ID. 19 |
| 35 | 0,63 | GGGGCATCCCGCTTGGCACAATGCGTGCGCGGGTA | SEQ. ID. 20 |
| 35 | 0,57 | GGGCCATCCGTTTGGCACAATGCGTGCGCGGATA | SEQ. ID. 21 |
| 34 | 0,38 | GGGGGACCGCCTGTAACGG**T**GGGTTCGGCGCGA | SEQ. ID. 22 |
| 34 | 0,28 | GCGGGAGCGCGCGGACCG**AT**GGGT**T**CCGGGGTA | SEQ. ID. 23 |
| 34 | 0,35 | GGGGGAGCGCGCTGGACGCTGGGG**TT**CGCCGGGMA | SEQ. ID. 24 |
| 33 | 0,30 | GGGGGCCGCCGCCTGGAACGA**TT**CGGGGTGA | SEQ. ID. 25 |
| 33 | 0,29 | GGGGGOCGCC**T**GGAACGATG3GTTCGGTGCG**A** | SEQ. ID. 26 |
| 33 | 0,29 | GTGGGGACCGCCT**G**GGAACGATGGGTCGGCGCT**A** | SEQ. ID. 27 |
| 33 | 0,31 | GT**A**GGCACCGCCCTGGAGGATGGGCTCGGCTGT**A** | SEQ. ID. 28 |
| 33 | 0.26 | G**T**CGGGGGGGGTCGG**A**GCGCGCC**A**TCACAGGTGGTGGA | SEQ. ID. 29 |
| 34 | 1,14 | TACCCT**A**GCTCAAGCGC**AT**GCGGCGGCGTCCC**TG**C | SEQ. ID. 30 |
| 35 | 10,00 | TCCACCCCGACCACTCAAGGGAA**CCGGT**CGGCCCGCGC | SEQ. ID. 31 |
| 33 | 1,67 | **T**CGCGCC**A**ACCC**A**TCGTTCCAGGCGCGTCCCCC | SEQ. ID. 32 |

| | | | |
|---|---|---|---|
| [Captions: Nucleotídeos = Nucleotides; indice de Presença = Presence Index; Sequêncian = n sequence; Nome = Name] | | | |

The polynucleotides of Table 1 were subjected to the search for similarities in the PUBMED BLAST tool and the result forthis search showed no native counterpart, since in the Blast-n mode only parts of the sequences were found, and none of the possible full sequences 5'-CONS-SEQ.ID.n-CONS-3' (I) has been identified as natural, and considering MEGABLAST that tends to search for the entire sequence, none of the sequences presented its natural counterpart.

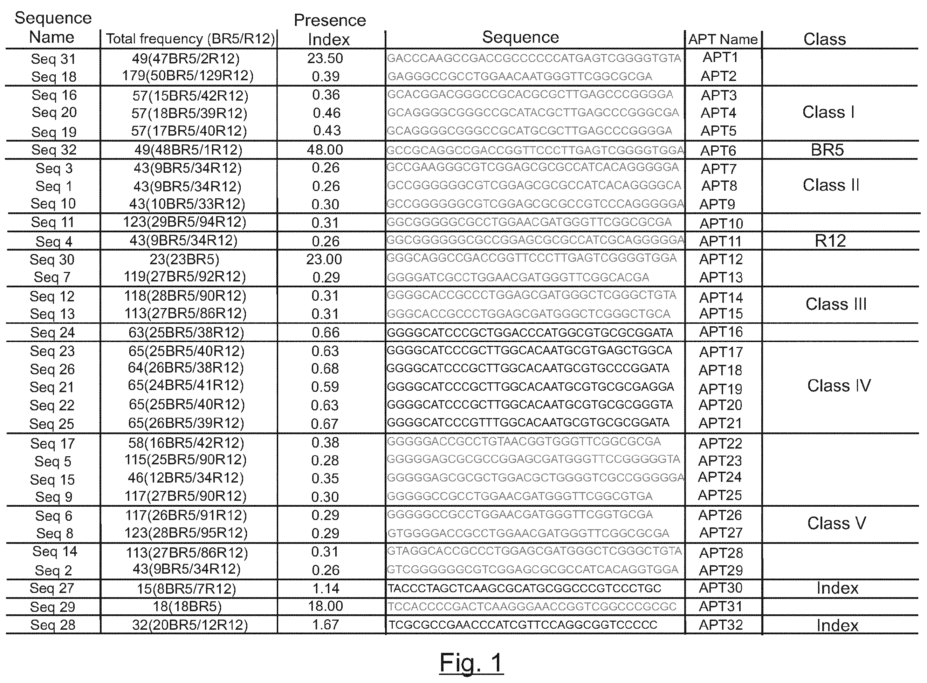

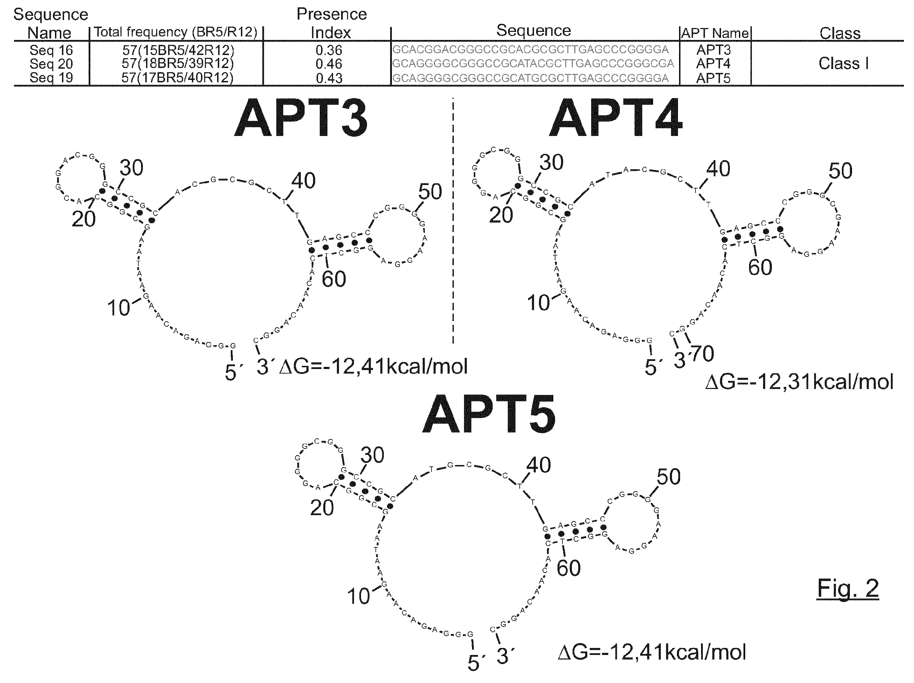

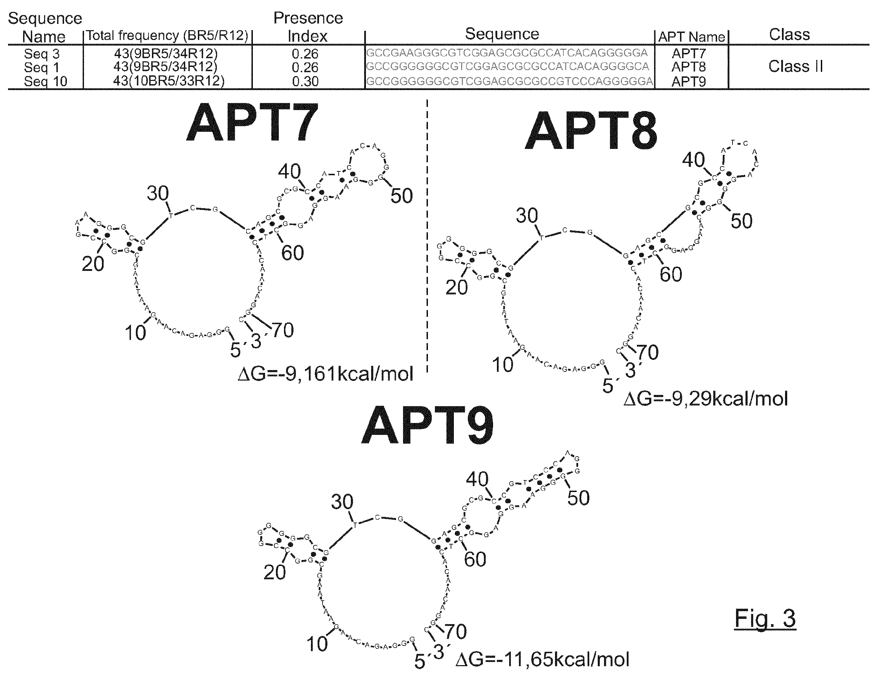

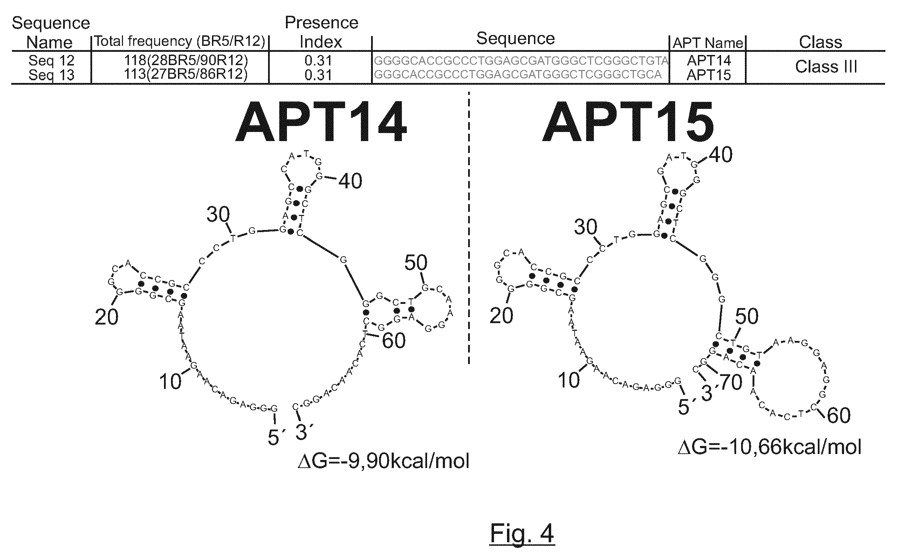

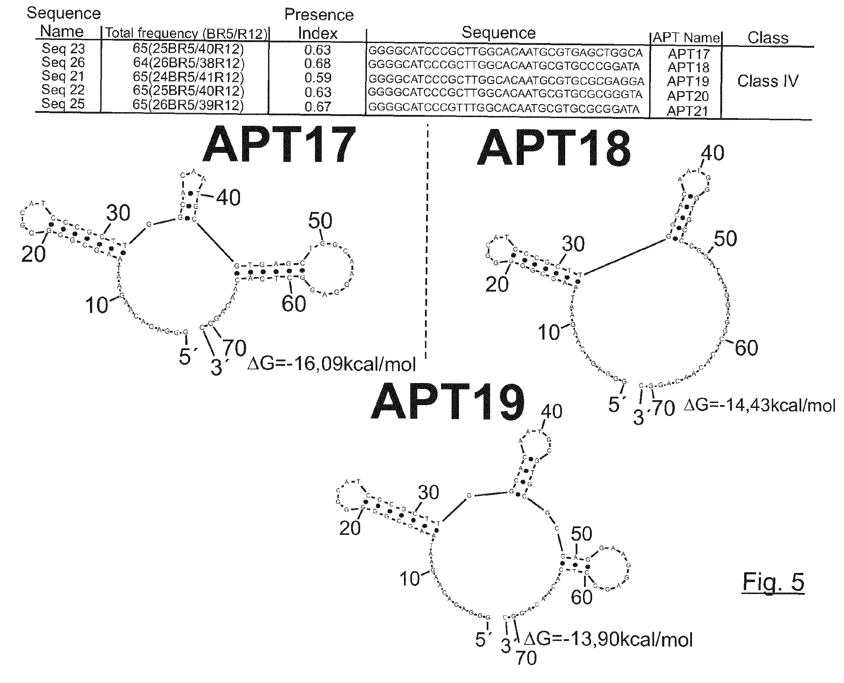

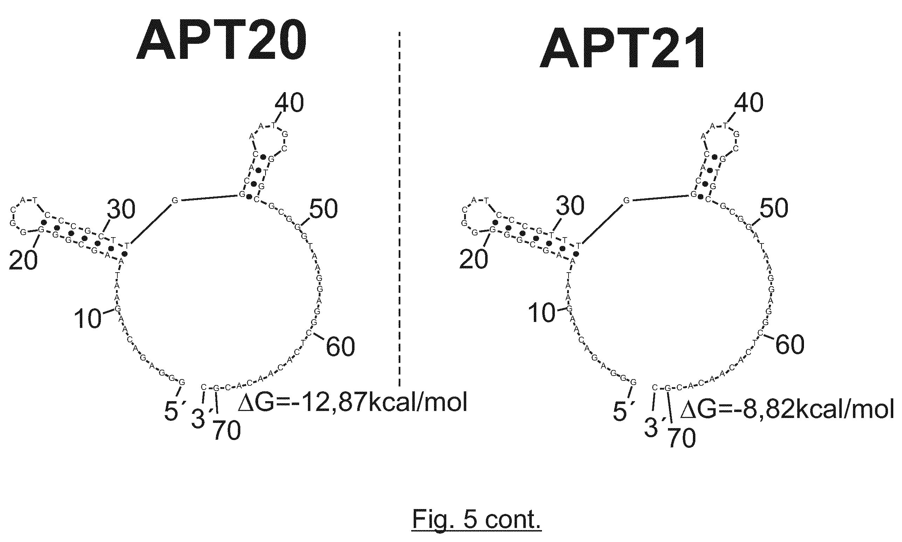

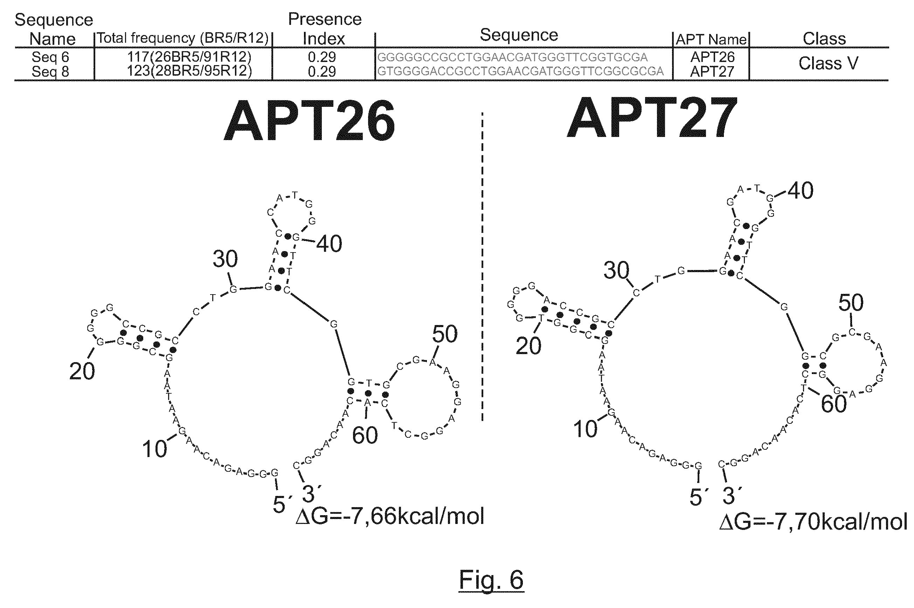

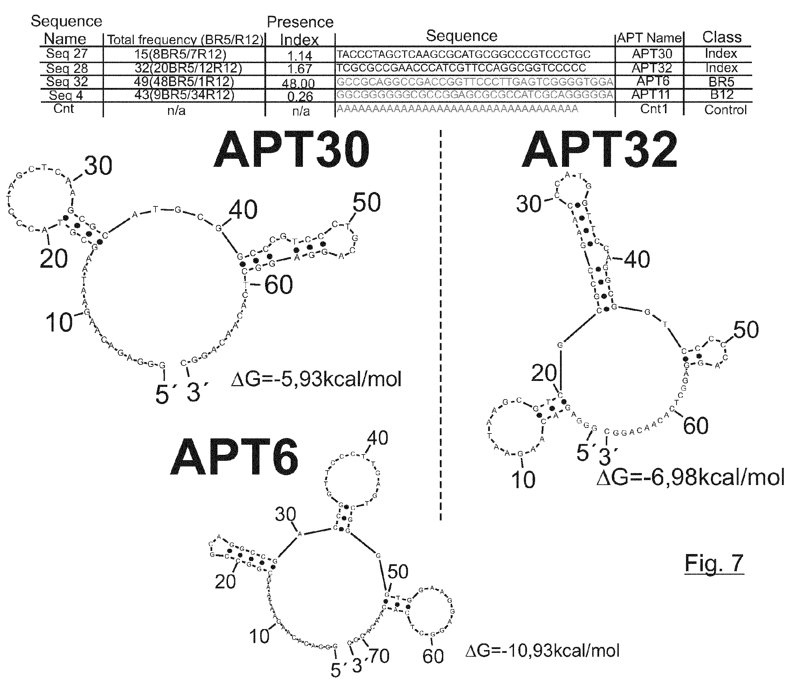

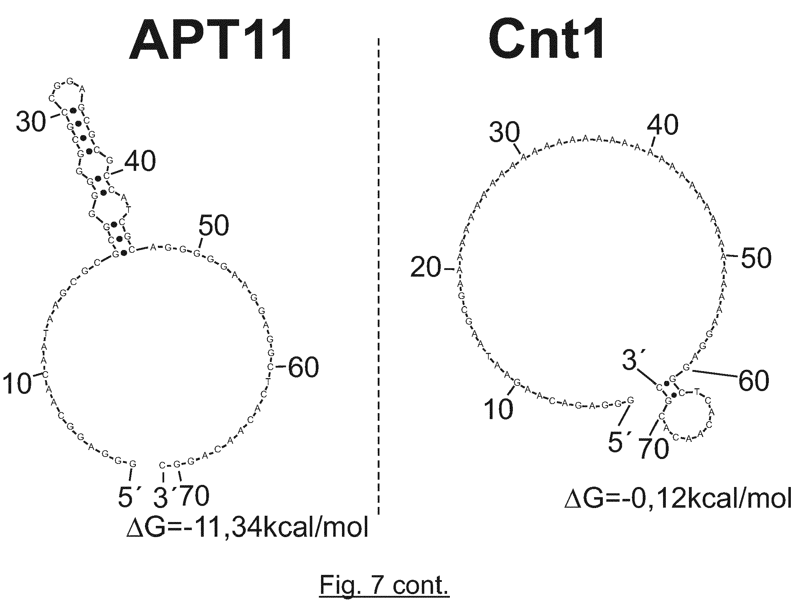

From the sequences identified in table1, the chemically-modified polynucleotides of formula (I) 5'-CONS SEQ.ID.n-CONS-3 ' (I) obtained were grouped into classes of molecules according to the similarities of sequence motifs in the previously random region (Figure 1). Within these classes, one can see that besides having similar sequences, molecules also share high structural conservation as shown in Figures 2 to 7.

The sequences in bold are those that showed a similar presence index in the two libraries analyzed. It is noticed that in the alignment by similarity, five classes of polynucleotides were formed (I, II, III, IV and V) and four other sequences were evidenced, two with presence indices of 0.5 to 1.5 that do not have similarity with any other, and two sequences that had the highest and the lowest presence index, indicating strong presence in BR5 and R12, respectively. In blue one can notice which sequences were selected to synthesize and follow the experiments with isolated polynucleotides, which in turn were named APTL-32 according to the alignment process described above.

The last object of the present invention relates to a process for the separation of stem cells present in the lipoaspirate comprising the use of polynucleotides of formula (I) classes I to V, in the identification of stem cells. As the chemically-modified polynucleotides of formula (I) have affinity for the marker molecules present in the membrane of stem cells, the polynucleotides of formula (I) exhibit greater affinity for said marker molecules, bind to them making possible the identification of these cells by the cell identification devices known to the person skilled in the art, such as a FACS machine, and others.

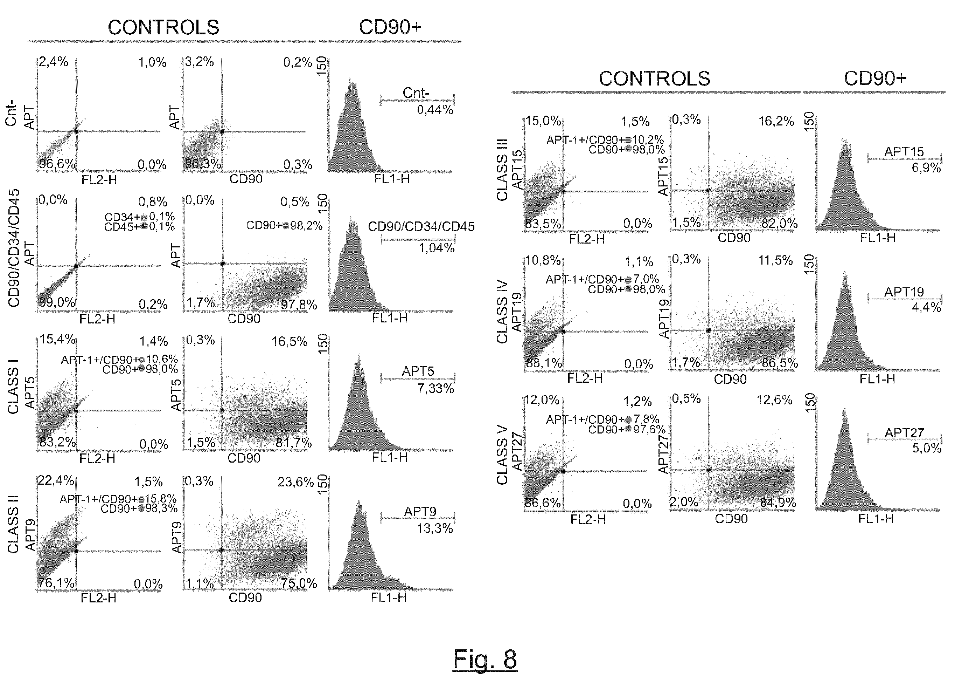

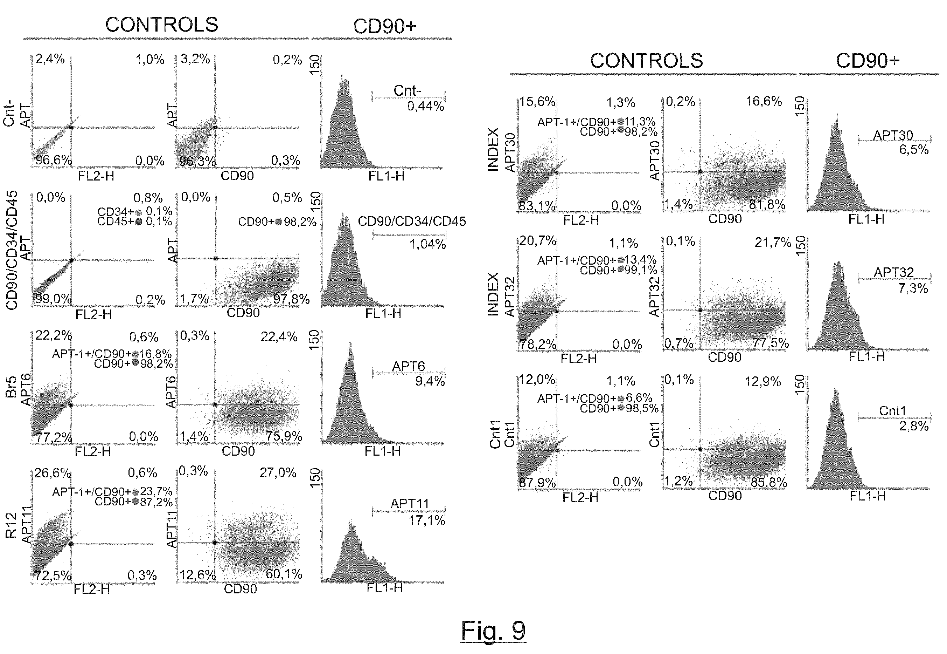

Figures 8 and 9 show that the chemically-modified polynucleotides of formula (I) whose random sequence corresponds to SEQ. ID. 4 and 10 alone are able to accurately identify a large percentage of stem cells. In Figure 8, the captions mean: Cells "(■). Negative control (Cnt-) are experiments for determining the marking profiles without antibody or chemically-modified polynucleotides of formula (I); CD90/CD34/CD45 is the standardization of marking for these molecular markers, wherein CD34 (■) and CD45 (■) have the FL-2 axis, and are represented together, because it is known that they do not mark stem cells from the lipoaspirate. The marking of stem cells from the lipoaspirate for CD90 (■) was 98.0% of events marked, making it impossible to evaluate the events within the cell region. From that point, the evaluation of each one of the polynucleotide classes of formula (I) found was carried out (I) for the verification of which class has the highest efficiency in marking cellular sub-groups; and the marked cells are obtained only by CD90 (■) and APTx/CD90 (■).

Figure 9 shows an experiment wherein:the events are "cells" (■). The negative control (Cnt-) is determined by marking profiles without antibody or chemically-modified polynucleotides of formula (I); CD90/CD34/CD45 has the marking standardization for these molecular markers, wherein CD34 (■) and CD45(■) have the FL-2 axis, and are represented together, because it is known that they do not mark stem cells from the lipoaspirate. The marking of stem cells from the lipoaspirate for CD90 (■) was 98.2% of marked events making it impossible to evaluate the events within the cell region. From that point, an evaluation was made of each of the classes of chemically-modified polynucleotides of formula (I) found, wherein it was possible to assess which ones have greater efficiency in marking cellular sub-groups; and the marked cells are obtained only by CD90 (■) and APTX/CD90 (■).

Thus, as noted, the polynucleotide 5'-CONS-SEQ.ID.9-CONS-3'alone was able to identify 15.8% of the cells and the 5'-CONS SEQ.ID.11-CONS -3' alone identified 23.7% of isolated stem cells from the lipoaspirate. Thus indicating that this is probably the number of cells showing unique molecular signature and not shared with other somatic cells present in adipose tissue, and each of the tested polynucleotide may be binding to different targets and have different affinity constants.

Although the present invention and the best modes of execution thereof have been described so that a person skilled in the art may carry it out and use it, it is understood and appreciated that there are equivalents to the sample embodiments described in the present application and that modifications and variations may be carried out without deviating from the scope and spirit of the inventions, which should not be limited by exemplified embodiments.

## Claims

1. Chemically-modified polynucleotides **characterized by** having the formula (I):
5'-CONS-SEQ.ID.n-CONS-3' (I), wherein:
5' CONS is one of the identifying sequences SEQ. ID. 33 and/or SEQ. ID. 34; and
CONS-3' is one of the identifying sequences SEQ. ID. 33 and/or SEQ. ID. 34;
SEQ.ID.n is alternately or separately one or more of identifying sequences of SEQ. ID. 1 to SEQ. ID. 32 containing one or more modified pyrimidines and at least one inverted nucleotide.

2. Polynucleotides, according to claim 1, **characterized by** in formula (I):
5'-CONS is SEQ. ID. 33;
CONS-3' is SEQ. ID. 34;
SEQ.ID.n is separately each one of the identifying sequences of SEQ. ID. 1 to SEQ. ID. to 32 containing one or more modified pyrimidines and the last inverted nucleotide.

3. Polynucleotides, according to claims 1 or 2 **characterized by** modifications in one or more pyrimidines to be made by the substitution of 2-OH with a halogen atom.

4. Polynucleotides, according to claim 1, 2 or 3, **characterized in that** the modified pyrimidines are 2'F-dCTP and 2'F-dCUTP.

5. Polynucleotides, according to claim 1 or 2, **characterized by** having high binding affinity for mesenchymal stem cells from human adipose tissue and any other of mesenchymal origin.

6. Polynucleotides, according to claim 1 or 2, **characterized by** being for the detection and identification of mesenchymal stem cells, preferably human mesenchymal cells.

7. A method of producing the chemically-modified polynucleotides of formula (I):
5'-CONS-SEQ.ID.n-CONS-3' (I) which comprises the steps of:
(a) Producing a polynucleotide library;
(b) Presenting polynucleotides to possible binding targets;
(c) Selecting and amplifying the ligands; and
(d) Modifying the ligands.

8. The method according to claim 7, **characterized in that** in step (a) the primer polynucleotides of sequences of SEQ. ID. 33 and SEQ. ID. 34 are used to produce an ssDNA library containing about 10²⁰ polynucleotides encoding sequences containing a randomized region flanked by constant regions represented by polynucleotides that pair with primer sequences SEQ. ID. 33 and SEQ. ID. 34.

9. A method, according to any claims 7 or 8, **characterized by** incubating the polynucleotides of formula (I) in a selection buffer and possible targets, and those that bind or remain bound after washing in each washing cycle, being collected and passed directly to the steps of enzymatic amplification through DNA polymerase chain reaction (PCR).

10. The method according to claim 9, **characterized in that** the targets of the polynucleotides of formula (I) 5'-CONS-SEQ.ID.n-CONS-3' are organic molecules of molecular signature of mesenchymal stem cells membrane.

11. The method according to claim 7, **characterized in that** step (c) occurs randomly, the polynucleotides of the ssDNA library are amplified; the reaction occurs in cycles of between 4 to 6 minutes at a temperature between 90° to 100°C; followed by 4 to 6 minutes at a temperature between 40° and 45°C and 10 minutes at 72°C.

12. The method according to claim 11, **characterized in that** after the double-strand synthesis, the primer of SEQ. ID. 34 is added conferring a triple marker tail of biotin.

13. The method, according to any claims 7 or 12, **characterized in that** the amplification proceeds for about 1 to 5 minutes at a temperature of between 90° and 100°C to separate the strands and cycles with 30 to 60 seconds at a temperature between 90° and 100°C, followed by from 30 to 60 seconds at a temperature between 40° to 50°C and a step of 1 to 5 minutes at a temperature between 70° to 75°C, and this cycle is repeated 20 to 30 times before a final extension step of 5 to 12 minutes, at a temperature between 70° to 75°C.

14. The method, according to any claims 7, 8, 9, 10 or 12, **characterized in that** the binding event sequence to the target, selection and amplification is a SELEX cycle.

15. The method according to claim 7, **characterized in that** the polynucleotides of formula (I) 5'-CONS-SEQ.ID.n -CONS-3' are obtained, wherein SEQ.ID.n is independently and randomly a sequence among SEQ. ID. 1 to 32.
